## Europäisches Patentamt
## European Patent Office
## Office européen des brevets

(11) Veröffentlichungsnummer: **0 039 858**
**B1**

(12) **EUROPÄISCHE PATENTSCHRIFT**

(45) Veröffentlichungstag der Patentschrift:
**30.11.83**

(51) Int. Cl.³: **C 14 C 9/02**

(21) Anmeldenummer: **81103327.3**

(22) Anmeldetag: **02.05.81**

(54) Verfahren zur Herstellung von Fettungsmitteln für Leder und Pelze.

(30) Priorität: **12.05.80 DE 3018176**

(43) Veröffentlichungstag der Anmeldung:
**18.11.81 Patentblatt 81/46**

(45) Bekanntmachung des Hinweises auf die Patenterteilung:
**30.11.83 Patentblatt 83/48**

(84) Benannte Vertragsstaaten:
**AT BE CH DE FR GB IT LI LU NL**

(56) Entgegenhaltungen:
**DE - B - 1 125 105**
**DE - C - 2 245 077**
**FR - A - 2 031 167**
**GB - A - 1 166 725**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Willmund, Wolf-Dieter, Dr., Einsteinstrasse 9, D-4000 Düsseldorf 13 (DE)**
Erfinder: **Plapper, Jürgen, Dr., Kalstert 156, D-4010 Hilden (DE)**
Erfinder: **Pieper, Friedrich, Zehnten Weg 10, D-4018 Langenfeld (DE)**
Erfinder: **Heidrich, Jochen, Dr., Robert-Koch-Strasse 41, D-4019 Monheim (DE)**

Verfahren zur Herstellung von Fettungsmitteln für Leder und Pelze

Gegenstand des Patentes DE-C-2 245 077 sind Fettungsmittel für Leder oder Pelze auf der Basis von sulfonierten Chlorierungsprodukten von natürlichen oder synthetischen höheren Fettsäuren oder Fettsäureestern in Form ihrer Alkali-, Ammonium- oder Aminsalze, dadurch gekennzeichnet, daß sie aus solchen sulfonierten Chlorierungsprodukten bestehen, die durch Chlorieren von höheren Fettsäuren oder von Estern höherer Fettsäuren der Kettenlängen $C_8$ bis $C_{24}$ bis zu einem Chlorgehalt von 20 bis 45 Gewichtsprozent, wobei die Chlorierungsprodukte im wesentlichen keine olefinischen Doppelbindungen mehr enthalten, und nachfolgende Sulfonierung mit $SO_3$ bis zu einem Gehalt von 40 bis 100 Molprozent $SO_3$, bezogen auf Chlorierungsprodukt, erhalten worden sind.

Es wurde nun gefunden, daß man in einer einstufigen Reaktion zu wirksamen sulfonierten Chlorierungsprodukten für die Fettung von Leder und Pelzen kommen kann, wenn man höhere Fettsäuren oder Fettsäureester einer Sulfochlorierungsreaktion mit Chlor und Schwefeldioxid unterwirft.

Gegenstand der Erfindung ist somit ein Verfahren zur Herstellung von Fettungsmitteln für Leder oder Pelze, dadurch gekennzeichnet, daß man höhere Fettsäuren oder Ester höherer Fettsäuren der Kettenlängen $C_8$ bis $C_{24}$ mittels Chlor und $SO_2$ ggf. unter UV-Bestrahlung bei $20-90°C$ bis zu einem Gehalt an organisch gebundenem Chlor von $5-30$ Gewichtsprozent und einem Gehalt an $SO_2Cl$-Gruppen von $1-20$ Gewichtsprozent sulfochloriert, wobei das Verhältnis von Chloratomen zu $SO_2Cl$-Gruppen $0,7:1$ bis $70:1$ beträgt, und nachfolgend verseift.

Bisher war man der Ansicht, daß sich die Sulfochlorierungsreaktion mit Erfolg nur auf Paraffine und Cycloparaffine anwenden läßt (Römpps Chemie-Lexikon, 7. Auflage, 3398/ 3399). Die Sulfochlorierung aller sauerstoffhaltigen Produkte sollte dagegen ungünstig verlaufen (Lindner, Tenside, Textilhilfsmittel, Waschrohstoffe (1964), Band I, S. 714). Es ist daher überaus überraschend, daß die Anwendung des Sulfochlorierungsverfahrens auf höhere Fettsäuren oder Ester höherer Fettsäuren unter Einhaltung der im Patentanspruch angegebenen Bedingungen zu wirksamen Leder- und Pelzfettungsmitteln führt, deren Wirksamkeit diejenige der Produkte des Patentes DE-C-2 245 077 noch übertrifft.

Zur Herstellung der beanspruchten Fettungsmittel geht man vorzugsweise von natürlich vorkommenden höheren Fettsäuren oder von Estern höherer Fettsäuren der Kettenlängen $C_8$ bis $C_{24}$, vorzugsweise $C_{10}$ bis $C_{20}$ aus. Bevorzugt werden Gemische von Fettsäuren oder von Fetten oder Ölen, wie sie in natürlich vorkommenden Fettstoffen vorliegen, insbesondere solchen mit einem Anteil an einfach oder mehrfach ungesättigten Fettsäuren. Beispiele hierfür sind: Kokosöl, Sojaöl, Palmkernöl, Baumwollsaatöl, Rüböl, Leinöl, Rizinusöl, Sonnenblumenöl, Olivenöl, Klauenöl, Erdnußöl, Heringstran, Dorschtran, Haifischtran, Waltran, Talgfette oder Schweineschmalz, ferner die aus diesen Fetten oder Ölen gewonnenen Fettsäuregemische, ferner natürlich vorkommende Wachsester, z. B. Spermöl. Es eignen sich jedoch auch solche Fettstoffe, die keine ungesättigten Fettsäuren enthalten oder deren Gehalt an ungesättigten Fettsäuren verringert worden ist, z. B. durch Abpressen, Auskristallisieren oder Destillation gewonnene gesättigte Fette oder partiell oder vollständig gehärtete Fette oder Öle.

Weiterhin kommen als Ausgangsstoffe für die Herstellung der Fettungsmittel auch synthetisch hergestellte Ester aus gesättigten oder ungesättigten Fettsäuren der Kettenlängen $C_8$ bis $C_{24}$, vorzugsweise $C_{10}$ bis $C_{20}$ in Betracht, wie Decancarbonsäure, Palmitinsäure, Stearinsäure, Behensäure, Dodecencarbonsäure, Ölsäure, Linolsäure oder durch Paraffinoxidation hergestellte Carbonsäuren, mit ein- oder mehrwertigen aliphatischen Alkoholen der Kettenlängen $C_1$ bis $C_6$, wie Methanol, Äthanol, Isopropanol, Butanol, Äthylenglykol, 1,2-Propylenglykol, Glycerin, Pentaerythrit oder Sorbit, oder höheren Alkoholen der Kettenlängen $C_8$ bis $C_{24}$, wie Decylalkohol oder Oleylalkohol.

Wegen ihrer leichten Zugänglichkeit werden als Ausgangsstoffe die natürlichen tierischen oder pflanzlichen Fette, Öle oder Wachse sowie die daraus hergestellten Umesterungsprodukte mit niederen einwertigen aliphatischen Alkoholen, insbesondere Methylalkohol, sowie die entsprechenden Fettsäuregemische bevorzugt.

Die genannten Ausgangsstoffe werden in an sich bekannter Weise sulfochloriert durch gleichzeitiges Einleiten von $Cl_2$ und $SO_2$ im Molverhältnis von $85:1$ bis $1,4:1$. Die Reaktionstemperatur beträgt $20-90°C$ und wird gegebenenfalls durch Kühlen auf den gewünschten Stand gehalten. Bevorzugt wird eine Reaktionstemperatur von $40-75°C$. Durch Bestrahlung mit UV-Licht (Quecksilberdampf-Lampe) wird der Reaktionsablauf gefördert. Die Reaktion ist nach etwa $2-10$ Stunden beendet, nach welcher Zeit $5-30$ Gewichtsprozent Chlor und $1-20$ Gewichtsprozent $SO_2Cl$-Gruppen angelagert worden sind. Das Verhältnis von Chloratomen zu $SO_2Cl$-Gruppen liegt bei $0,7:1$ bis $70:1$. Die nachfolgende Verseifung wird mit wäßriger, etwa 30%iger Natrium- oder Kaliumhydroxidlösung bei etwa $50°C$ durchgeführt, die Neutralisation erfolgt entweder mit einem Überschuß der gleichen Alkalihydroxyde oder mit Ammoniaklösung oder mit einem aliphatischen oder cycloaliphatischen Amin oder einem Alkanolamin der Kettenlängen $C_2$ bis $C_6$, wie Triäthanolamin. Man erhält flüssige hochkonzentrierte wasseremulgierbare Produkte mit ausgezeichneter Oxidations-, Licht- und Säurestabilität, die sich für die Fettung aller hellen, pastellgefärbten und weißen Leder sowie zur Fettung auch wertvoller und empfindlicher Pelze hervorragend eignen.

Bei Verwendung dunkelfarbiger oder stärker ungesättigter Ausgangsstoffe kann sich eine Bleichung der Sulfonierungsprodukte empfehlen. Diese wird in üblicher Weise durch Zugabe geringer Mengen von etwa 0,5 bis 5%, vorzugsweise 1 bis 4% $H_2O_2$ zum sauren Sulfonierungsprodukt durchgeführt, wobei die Temperaturen zwischen 20 und 80, vorzugsweise zwischen 40 und 60° C liegen. Durch diese Maßnahme können auch dunkelfarbige Sulfonierungsprodukte sehr weitgehend aufgehellt werden.

Die Produkte werden in üblicher Weise in Form wäßriger Emulsionen zum Lickern von Leder oder zur Pelzbehandlung angewendet. Die Produkte sind selbstemulgierend, so daß weitere Zusätze an Emulgatoren im allgemeinen nicht notwendig sind. Zur Erzielung spezieller Effekte können die Sulfonierungsprodukte jedoch mit den entsprechenden nicht sulfonierten Chlorierungsprodukten, oder anderen üblichen Lederbehandlungsmitteln kombiniert werden, wie z. B. nichtsulfonierte Öle oder Fette, wie Fischtran, Spermöl, Klauenöl und dergleichen, oder synthetische Fettungsmittel, wie Chlroparaffine, Paraffinsulfonate, sulfierte native Fette oder Öle oder synthetische Fettsäureester, oder Mineralöle oder dergleichen, gegebenenfalls in Verbindung mit anionischen, nichtionogenen oder kationischen Emulgatoren, wie Ethylenoxidanlagerungsprodukte an höhere Fettalkohole, Alkylphenole oder Fettamine der Kettenlängen $C_{10}$ bis $C_{20}$. Eine Stabilisierung der Produkte kann durch Unschädlichmachen evtl. noch vorhandener oder neu gebildeter Chlorwasserstoffreste mittels Epoxidverbindungen in Mengen von 0,5 bis 5 Gewichtsprozent erreicht werden. Beispiele hierfür sind: Glyzid, Epichlorhydrin, Glyzidylether ein- oder mehrwertiger Alkohole, wie Glykol, Glycerin oder Sorbit, sowie epoxidierte Fettstoffe, z. B. epoxiertes Sojaöl, Leinöl oder Ölsäurebutylester.

Die Produkte werden vom Leder gut aufgenommen und geben ausgezeichnete Fettungs- und Weichmachungseffekte, die eine bemerkenswerte Beständigkeit gegen Wasser und wäßrige oder organische Reinigungsmittellösungen besitzen. Ihre Neigung zum Wandern bei thermischer Beanspruchung ist sehr gering, so daß sich z. B. Verklebungsvorgänge oder das Anvulkanisieren von Gummisohlen an Schuhoberteile ohne Schwierigkeiten durchführen lassen. Besonders hervorzuheben ist die gute Licht-, Oxidations- und Säurebeständigkeit der Produkte, die sie auch zur Fettung empfindlicher und heller Leder und Pelze geeignet macht. Die behandelten Leder oder Pelze zeichnen sich durch einen besonders angenehmen weichen und schmalzigen Griff sowie einen schönen Glanz des Pelzhaares aus.

Die in den nachfolgenden Beispielen beschriebene Sulfochlorierungsreaktion wurden in der folgenden Apparatur durchgeführt:

Auf einem 2-l-Rundkolben mit Bodenablaß steht ein Reaktorturm aus Glas, der mit Raschigringen gefüllt ist und der einen Doppelmantel für eine Heiz- bzw. Kühlflüssigkeit besitzt.

Das Einsatzmaterial wird durch den Bodenablaß des Rundkolbens mittels einer Schlauchpumpe durch ein beheizbares Steigrohr in den Kopf des Reaktionsturmes gepumpt.

Die Gase (Chlor und Schwefeldioxid) werden am unteren Ende des Turmes über Nadelventile eingeleitet. Die Dosierung wird mittels Rotametern durchgeführt. Die Strömungsgeschwindigkeit der Gase liegt für Chlor zwischen 25 und 120 l/h und für $SO_2$ zwischen 4 und 50 l/h.

Das entstandene HCl-Gas wird mit dem Rest an nicht umgesetzten Ausgangsgasen am Kopf des Reaktionsrohres über ein Waschflaschensystem entnommen. Die gläserne Reaktionskolonne wird von außen mit einer Hg-Dampflampe bestrahlt.

## Beispiel 1

1000 g Talgfettsäuremethylester ($C_{16}$—$C_{18}$) wurden im Reaktor auf 45° C geheizt und mittels einer Schlauchpumpe mit ca. 1 l/h umgepumpt.

Im Gegenstrom wurde Chlor (40 l/h) und $SO_2$ (7 l/h) in den von außen mit einer Hg-Dampflampe belichteten Reaktor eingeleitet. Die Reaktion lief spontan ab, was durch Bildung von HCl-Nebeln sichtbar wurde.

Die Reaktion wurde nach 220 min beendet und das Reaktionsprodukt (1350 g) im Vakuum von den gelösten Gasen (hauptsächlich HCl) befreit. Es enthielt 17 Gew.-% organisch gebundenes Chlor und 7,7 Gew.-% $SO_2Cl$-Gruppen.

Die Verseifung und Neutralisation wurde mit 300 g wäßriger 30%iger NaOH bei Temperaturen von 40—50° C durchgeführt. Dabei entstand eine stabile Emulsion, die ca. 40% Chlor-Talgfettsäuremethylester-sulfonat, 42% Chlor-Talgfettsäuremethylester und 18% anorganische Salze und Wasser enthielt.

## Beispiel 2

Bei der beschriebenen Apparatur wurden 1000 g Kokosfettsäuremethylester ($C_{12}$—$C_{18}$) innerhalb von 3,5 Stunden bei 40° C unter UV-Bestrahlung mit 455 g Chlor (41,5 l/h) und 90 g $SO_2$ (9 l/h) umgesetzt. Nach Entfernen der gelösten Gase, insbesondere HCl unter Vakuum wurden 1319 g Sulfochlorierungsprodukt mit einem Gehalt von 14,4 Gew.-% Chlor und 8,5 Gew.-% $SO_2Cl$ erhalten.

1151 g des Reaktionsproduktes wurden mit 268 g einer 31%igen Natriumhydroxidlösung verseift und neutralisiert. Das homogenisierte Produkt enthielt ca. 50 Gew.-% Chlorkokosfettsäuremethylester-sulfonat, 30 Gew.-% Chlorkokosfettsäuremethylester und 20 Gew.-% anorganische Salze und Wasser. Das Produkt ließt sich mit Wasser zu einer stabilen Emulsion verdünnen.

### Beispiel 3

In der beschriebenen Apparatur wurden 1000 g Palmkernöl bei 50°C unter UV-Bestrahlung innerhalb von 5 Stunden mit 410 g Chlor (26 l/h) und 123 g $SO_2$ (8,6 l/h) umgesetzt. Das erhaltene Produkt wurde im Vakuum entgast. Die Ausbeute betrug 1340 g Sulfochlorierungsprodukt mit einem Gehalt an 13,4 Gew.-% Chlor und 10,5 Gew.-% $SO_2Cl$.

1166 g des Reaktionsproduktes wurden mit 421 g 31%iger wäßriger Natriumhydroxidlösung bei 50°C verseift und neutralisiert. Es entstand eine stabile Emulsion mit einem Gehalt von ca. 42 Gew.-% Chlor-Palmkernöl-sulfonat, 30 Gew.-% Chlor-Palmkernöl und 28 Gew.-% anorganischen Salzen und Wasser.

### Beispiel 4

In der gleichen Apparatur wurden 1000 g Fancy-Talg ($C_{16}-C_{18}$) bei 50°C unter UV-Bestrahlung innerhalb von 5,5 Stunden mit 440 g Chlor (25,5 l/h) und 130 g $SO_2$ (8,5 l/h) umgesetzt. Nach dem Entgasen erhielt man 1322 g des Sulfochlorierungsproduktes mit einem Gehalt von 15,4 Gew.-% Chlor und 9,7 Gew.-% $SO_2Cl$.

980 g dieses Produktes wurden mit 261 g 31%iger Natriumhydroxidlösung bei 50°C hydrolysiert und neutralisiert. Es entstand eine stabile Emulsion, die ca. 47 Gew.-% Chlor-Talg-sulfonat, 31 Gew.-% Chlor-Talg und 22 Gew.-% anorganische Salze und Wasser enthielt.

### Beispiel 5

Ein mit synthetischen oder vegetabilen Gerbstoffen oder Harzgerbstoffen oder mit einer Kombination der genannten Gerbstoffe nachgegerbtes Schuhoberleder wurde im Faß bei 60°C 45 Minuten mit 100—120% Flotte und 5 bis 6% des Sulfochlorierungsproduktes nach Beispiel 2 als fettende Substanz, auf Leder bezogen, gelickert. Die in üblicher Weise getrockneten und fertiggestellten Leder zeichneten sich durch weichen, geschmeidigen und vollen Griff, gute Narbenfestigkeit sowie ausgezeichnete Licht- und Oxidationsbeständigkeit aus.

### Beispiel 6

Chromgegerbtes und gefärbtes Bekleidungsleder wurde bei 60°C 45 Minuten mit 100—120% Flotte, 7—10% fettender Substanz, bestehend aus einem Gemisch von 80 Gew.-% des Sulfochlorierungsproduktes nach Beispiel 1 und 20 Gew.-% eines entsprechenden nicht sulfonierten Chlor-Talgfettsäuremethylesters, im Faß gelickert.

Die in üblicher Weise getrockneten und fertiggestellten Leder zeichneten sich durch weichen, geschmeidigen und vollen Griff sowie durch ausgezeichnete Licht- und Oxidationsstabilität aus.

### Beispiel 7

Pastellgefärbte, chromgegerbte Handschuhleder aus Lammfellen wurden bei 60°C 45 Minuten mit 100% Flotte und 6—8% fettender Substanz, bestehend aus einem Gemisch aus 75% des Sulfochlorierungsproduktes nach Beispiel 3, 21% eines nicht sulfierten Chlor-Talgfettsäuremethylesters und 4% eines mit 4 Mol Ethylenoxid umgesetzten Talgamins im Faß gelickert, getrocknet und in üblicher Weise fertiggestellt.

Die Leder zeichneten sich durch zügigen, geschmeidigen, weichen Griff und gute Lichtbeständigkeit aus.

### Beispiel 8

In üblicher Weise gepickelte Rindsblößen wurden mit 8% eines handelsüblichen Chromgerbstoffes mit einem $Cr_2O_3$-Gehalt von 25% mit 100% Flotte gegerbt und im gleichen Bad mit 2% fettender Substanz, bestehend aus einem Gemisch aus 95% des Sulfochlorierungsproduktes nach Beispiel 4 und 5% eines mit 20 Mol Ethylenoxid umgesetzten Talgalkohols vorgelickert. Die in üblicher Weise nachgegerbten, gefärbten und mit 8—10% des Sulfochlorierungsproduktes nachgefetteten und getrockneten Möbelleder zeichneten sich durch einen geschmeidigen, weichen Griff und gute Lichtbeständigkeit aus.

### Patentansprüche

1. Verfahren zur Herstellung von Fettungsmitteln für Leder oder Pelze, dadurch gekennzeichnet, daß man höhere Fettsäuren oder Ester höherer Fettsäuren der Kettenlängen $C_8$ bis $C_{24}$ mittels Chlor und $SO_2$ ggf. unter UV-Bestrahlung bei 20—90°C bis zu einem Gehalt an organisch gebundenem Chlor von 5—30 Gewichtsprozent und einem Gehalt an $SO_2Cl$-Gruppen von 1—20 Gewichtsprozent sulfochloriert, wobei das Verhältnis von Chloratom zu $SO_2Cl$-Gruppen 0,7 : 1 bis 70 : 1 beträgt, und nachfolgend verseift.

2. Verfahren nach Anspruch 1, dadurch gekennzeichnet, daß man die Sulfochlorierung durch gleichzeitiges Einleiten von Chlor und $SO_2$ im Molverhältnis von 85 : 1 bis 1,4 : 1 bei einer Reaktionstemperatur von 40—75°C durchführt.

3. Verfahren nach Anspruch 1—2, dadurch gekennzeichnet, daß man bis zu einem Verhältnis von Chloratomen zu $SO_2Cl$-Gruppen wie 2 : 1 bis 20 : 1, vorzugsweise 3 : 1 bis 7 : 1 sulfochloriert.

4. Verfahren nach Anspruch 1 bis 3, dadurch gekennzeichnet, daß man bei der Sulfochlorierung von natürlichen Fetten und Ölen ausgeht, die sich von Fettsäuren der Kettenlängen $C_{10}-C_{20}$ ableiten.

5. Verfahren nach Anspruch 1 bis 3, dadurch gekennzeichnet, daß man bei der Sulfochlorierung von Methylestern von Fettsäuren der Kettenlängen $C_{10}-C_{20}$ ausgeht.

## Claims

1. A process for the production of stuffing agents for leather or skins, characterized in that higher fatty acids or esters of higher fatty acids having chain lengths of from $C_8$ to $C_{24}$ are sulfochlorinated with chlorine and $SO_2$ at 20 to 90° C, optionally in the presence of UV light, up to an organically bound chlorine content of from 5 to 30% by weight and an $SO_2Cl$-group content of from 1 to 20% by weight, the ratio of chlorine atoms to $SO_2Cl$-groups amounting to between 0.7 : 1 and 70 : 1, followed by hydrolysis.

2. A process as claimed in Claim 1, characterized in that sulfochlorination is carried out by the simultaneous introduction of chlorine and $SO_2$ in a molar ratio of from 85 : 1 to 1.4 : 1 at a reaction temperature of from 40 to 75° C.

3. A process as claimed in Claims 1 and 2, characterized in that sulfochlorination is carried out up to a ratio of chlorine atoms to $SO_2Cl$-group of from 2 : 1 to 20 : 1 an preferably from 3 : 1 to 7 : 1.

4. A process as claimed in Claims 1 to 3, characterized in that natural fats and oils derived from fatty acids having chain lengths of from $C_{10}$ to $C_{20}$ are subjected to sulfochlorination.

5. A process as claimed in Claims 1 to 3, characterized in that methyl esters of fatty acids having chain lenghts of from $C_{10}$ to $C_{20}$ are subjected to sulfochlorination.

## Revendications

1. Procédé de préparation de nourritures pour cuirs ou peaux, caractérisé en ce que l'on soumet des acides gras supérieurs ou esters d'acides gras supérieurs à des longueurs de chaînes de $C_8$ à $C_{24}$, à sulfochloration par le chlore et $SO_2$, éventuellement sous irradiation par de la lumière ultraviolette, à une température de 20 à 90°C, jusqu'à une teneur en chlore organique de 5 à 30% en poids et une teneur en groupes $SO_2Cl$ de 1 à 20% en poids, le rapport des atomes de chlore aux groupes $SO_2Cl$ étant de 0,7 : 1 à 70 : 1, puis on saponifie.

2. Procédé selon la rev. 1, caractérisé en ce que l'on effectue la sulfochloration par injection simultanée de chlore et de $3O_2$ dans un rapport molaire de 85 : 1 à 1,4 : 1, à une température de réaction de 40 à 75°C.

3. Procédé selon les revendications 1−2, caractérisé en ce que l'on sulfochlore jusqu'à un rapport de 2 : 1 à 20 : 1, de préférence de 3 : 1 à 7 : 1, entre les atomes de chlore et les groupes $SO_2Cl$.

4. Procédé selon les revendications 1 à 3, caractérisé en ce que l'on part à la sulfochloration de graisses et huiles naturelles dérivant d'acides gras à des longueurs de chaînes en $C_{10}-C_{20}$.

5. Procédé selon les revendications 1 à 3, caractérisé en ce que l'on part à la sulfochloration d'esters méthyliques d'acides gras à des longueurs de chaînes de $C_{10}-C_{20}$.